# NOUVEAU FASCICULE DE BREVET EUROPEEN

(11) **EP 0 817 621 B2**
(45) Date de publication et mention de la décision concernant l'opposition: **26.12.2007**
(45) Mention de la délivrance du brevet: 20.06.2001
(21) Numéro de dépôt: 96911002.2
(22) Date de dépôt: 29.03.1996
(51) Int. Cl.: A61K 9/70

(54) **COMPOSITION PHARMACEUTIQUE FILMOGENE POUR ADMINISTRATION TRANSDERMIQUE**
FILMBILDENDE PHARMAZEUTISCHE ZUSAMMENSETZUNGEN ZUR TRANSDERMALEN VERABREICHUNG
PHARMACEUTICAL COMPOSITION FOR TRANSDERMIC DELIVERY

(30) Priorité: 30.03.1995 FR 9503776
(43) Date de publication de la demande: 14.01.1998
(73) Titulaire: Sanofi-Aventis, 75013 Paris (FR)
(72) Inventeur: SAUNAL, Henry, F-34000 Montpellier (FR); ILLEL, Brigitte, F-34000 Montpellier (FR)
(74) Mandataire: Thouret-Lemaitre, Elisabeth
(86) Numéro de dépôt international: PCT/FR1996/000480
(87) Numéro de publication internationale: WO 1996/030000

(56) Documents cités:
- EP-A- 0 055 396
- EP-A- 0 055 397
- EP-A- 0 289 900
- EP-A- 0 319 964
- EP-A- 0 640 352
- WO-A-88/09185
- WO-A-94/13257
- WO-A-95/09195
- Int.Pharmaceutics(1979) 3 ,247-260
- DD 287 652 A5
- "Topical Drug Bioavailability,Bioequivalence & Penetration" (1993), Ch.13,pp.225-259

## Description

La présente invention se rapporte, d'une manière générale, à une nouvelle composition pharmaceutique pour administration transdermique.

Plus précisément, l'invention concerne une composition pharmaceutique pour administration transdermique, capable de former un film souple après séchage sur la peau ainsi qu'une matrice utilisable dans une telle composition pharmaceutique.

L'administration transdermique de principes actifs médicamenteux représente une technique séduisante, car non invasive, douée d'avantages certains tels qu'absence d'effets secondaires gastro-intestinaux ou d'altérations de la substance active par les enzymes hépatiques.

Pour être efficace, cette technique doit toutefois permettre une pénétration transcutanée du médicament sur une période prolongée et de manière suffisante pour atteindre des taux plasmatiques compatibles avec un traitement thérapeutique.

L'estradiol, ainsi que d'autres hormones, est une substance résorbable au travers de la peau moyennant une formulation adaptée.

Comme de faibles doses, de l'ordre de 50 à 120 pg/ml de plasma sont nécessaires pour une efficacité clinique par exemple pour le traitement de déficiences en oestrogènes, l'estradiol représente un principe actif de choix pour une application transdermique.

A ce jour, divers systèmes ou dispositifs pour ce type d'administration ont été proposés qui permettent d'introduire, dans le flux sanguin, des doses contrôlées de substances médicamenteuses en général et d'estradiol en particulier.

On connaît par exemple le dispositif d'administration transdermique appelé communément "patch" constitué d'un réservoir formé de matériaux plastiques synthétiques contenant le principe actif. Ce réservoir peut être recouvert, sur sa face en contact avec la peau, par une membrane microporeuse dont la perméabilité à la substance active régule sa diffusion et par conséquent son dosage.

En dépit des réelles possibilités offertes par ce dispositif, en particulier pour une application à l'estradiol, on peut lui préférer d'autres systèmes. En effet il est connu que le patch peut se décoller de la peau et, par ailleurs, présenter un aspect souvent inesthétique.

Des gels contenant l'estradiol ont également été proposés. Toutefois, cette forme pharmaceutique peut présenter certains inconvénients à l'usage, généralement un toucher collant désagréable pour le patient de même qu'une maîtrise difficile de la dose de principe actif administrée et un contrôle délicat de la surface de recouvrement.

D'autres systèmes ont également été rapportés qui aident à l'administration transdermique de principes médicamenteux .

A ce titre, on peut citer des compositions pulvérisables comportant notamment des polymères capables de former un film au contact de la peau et de relarguer le principe actif pour une administration transcutanée. Des compositions de ce type, décrites par exemple dans le brevet EP 0319555, comprennent un principe actif, une matrice polymérique formant un film souple après séchage, un solvant contrôlant le relargage de la substance active à savoir un sorbitane macrogollaurate, une paraffine, un diglycéride ou triglycéride d'acide gras à chaîne moyenne ou le carbonate de propylène ainsi qu'un solvant, pour la matrice, capable de s'évaporer sur la peau et enfin un agent propulseur permettant de pulvériser cette composition renfermée dans un dispositif approprié.

Une matrice constituée d'éthylcellulose y est cependant déconseillée en raison de sa tendance à obturer le système de pulvérisation.

Dans le cadre de la présente invention, on a expérimenté une composition pulvérisable, selon le brevet EP cité ci-dessus, renfermant 2% d'estradiol comme principe actif.

A la suite de tests pratiqués sur la peau de rat nu, on n'a enregistré cependant qu'un flux d'estradiol de l'ordre de 0,03 µg. h⁻¹. cm⁻² à l'équilibre ce qui laisse présager des performances et une efficacité assez limitées lorsqu'il s'agira d'appliquer ces compositions sur une surface cutanée réduite en vue d'un traitement thérapeutique.

En outre, des compositions telles que proposées par le susdit brevet, caractérisées par la présence d'un gaz propulseur par exemple un hydrocarbure halogéné, sont de plus en plus controversées suite aux risques potentiels qu'elles sont susceptibles de provoquer pour l'environnement.

Au surplus, les compositions du brevet EP 0319555, par la présence de dérivés polyméthacryliques, dégagent une odeur caractéristique assez désagréable pour le patient ou son entourage.

D'autres compositions pharmaceutiques pour administration topique contenant un principe actif, un solvant et divers autres ingrédients sont également connues.

A titre d'exemple, on peut citer le brevet EP 55396 qui décrit des compositions antimycotiques formées :
* d'un éther de cellulose
* de 2 à 10% d'un agent d'étalement tel que le myristate d'isopropyle ou le palmitate d'isopropyle
* de 1 à 8% d'un agent de solubilisation
* de 0,05 à 1% d'un principe actif
* et d'un solvant tel que l'isopropanol

Toutefois, ces compositions si elles peuvent être utilisées pour des applications topiques dermatologiques se révèlent totalement inadaptées pour une application par pulvérisation même après ajout de 10 à 40% d'un gaz propulseur comme recommandé, car elles apparaissent trop visqueuses et susceptibles d'amener divers inconvénients tels que bouchage du dispositif de pulvérisation.

On peut également citer le brevet EP 319964 décrivant des compositions antifongiques capables de former un film comprenant :
* de 0,1 à 1,5% de tolnaphtalate
* de 10 à 20% d'un copolymère méthacrylate de diméthylaminoéthyle / méthacrylate
* de 0,5 à 10% d'un ester d'acide gras
* un soivant de type alcool et éventuellement de 0,1 à 5% d'un dérivé cellulosique.

Cette composition ne samble pas non plus adaptée pour une pulvérisation. En outre, comme déjà évoqué précédemment, la présence de dérivés méthacryliques lui procure une odeur rédhibitoire.

WO 94/13257 concerne des lotions traitantes dermatologiques de type émulsion eau dans huile comprenant de l'eau, un émollient, un polyol, un polymère et un agent thérapeutique. Dans ce document, les polymères cellulosiques ne sont guère mentionnés.

Enfin, on peut signaler le brevet EP 289900 qui se rapporte à des compositions antibactériennes à usage topique comprenant :
* de 0,5 à 10% d'un principe actif antibactérien
* de 1 à 30% d'un polymère non hydrosoluble notamment l'éthylcellulose ou un copolymère de polyvinylpyrrolidone
* de 0,5 à 40% d'un plastifiant, généralement une huile essentielle, jouant également le rôle de promoteur d'absorption transcutanée
* de 50 à 95% d'un solvant tel que l'éthanol

Comme on le sait, les huiles essentielles sont constituées en grande majorité de dérivés terpéniques.

Dans le cadre de l'invention, on a expérimenté une composition analogue à celle décrite dans ce brevet, contenant notamment l'estradiol comme principe actif et le limonène, qui est un terpène, comme promoteur d'absorption transdermique. Toutefois, une telle composition n'a fourni que de très faibles flux de diffusion transcutanée de ce principe actif.

La recherche d'une composition permettant la diffusion transdermique de principes actifs médicamenteux, notamment l'estradiol, à partir d'une surface de recouvrement faible et à des taux compatibles avec un traitement thérapeutique tout en étant exempte des inconvénients rapportés précédemment reste d'un intérêt majeur.

Or, il a été découvert, de manière surprenante selon l'invention, qu'il est possible de fournir des compositions pharmaceutiques pour l'administration transdermique d'estradiol ou d'autres substances médicamenteuses, à partir d'un film formé au niveau de la peau, compositions dépourvues des inconvénients cités ci-dessus mais capables, à partir d'une zone de recouvrement réduite et contrôlable, de délivrer le principe actif dans le flux sanguin de façon régulière, continue et à des taux plasmatiques atteignant largement des seuils thérapeutiques.

Ainsi, l'invention a pour objet principal une composition pharmaceutique pour administration transdermique consistant en :
1) une matrice polymérique de relargage capable de former un film souple après séchage
2) un principe actif
3) un promoteur d'absorption transcutanée du principe actif
4) un solvant non aqueux physiologiquement acceptable capable de dissoudre la matrice de relargage, le principe actif et le promoteur d'absorption transcutanée ainsi que de s'éliminer rapidement par évaporation au contact de la peau.

Dans le présent contexte, aussi bien dans la description que dans les revendications, on entend par "principe actif" soit une substance médicamenteuse destinée, après administration, à provoquer une réponse préventive ou thérapeutique, soit une association de deux ou plusieurs substances de ce type.

La matrice polymérique est généralement choisie parmi des substances polymériques ou copolymériques capables à la fois de former un film souple après évaporation du solvant et de relarguer le principe actif.

Généralement, cette matrice est présente à raison de 0% à 6% du poids de la composition selon l'invention par exemple de 4% à 6% par exemple 5%. Préférentiellement, on utilise de 1 à 5% en poids de matrice notamment 5%.

Le choix de cette matrice se portera principalement sur des substances polymériques ou copolymériques solubles dans le solvant physiologique de manière à former une solution homogène.

Parmi les polymères ou copolymères capables de répondre aux critères ci-dessus, on retient des polymères ou copolymères cellulosiques notamment parce qu'ils présentent après séchage une résistance à l'abrasion et une stabilité mécanique appropriées. Pour cette raison, des matrices cellulosiques de ce type pourront être rincées à l'eau sans crainte de détérioration ou encore d'élimination du principe actif.

A titre d'exemple de tels polymères ou copolymères cellulosiques utilisables dans les compositions de l'invention, on peut citer l'éthylcellulose, l'acétate butyrate de cellulose, l'acétate propionate de cellulose ou une hydroxypropylméthylcellulose greffée ou non telle que l'hydroxypropylméthylcellulose acétate succinnate.

L'éthylcellulose représente toutefois le polymère cellulosique préféré et, en conséquence, la matrice polymérique de relargage de choix pour la formation d'un film souple au contact de la peau.

En conséquence, selon un autre de ses aspects, l'invention a pour objet une composition pharmaceutique pour administration transdermique consistant en :
1) une matrice polymérique de relargage capable de former un film souple après séchage, choisie parmi des polymères ou copolymères cellulosiques
2) un principe actif, en particulier l'estradiol
3) un promoteur d'absorption transcutanée du principe actif
4) un solvant non aqueux physiologiquement acceptable capable de dissoudre la matrice de relargage, le principe actif et le promoteur d'absorption transcutanée ainsi que de s'éliminer rapidement par évaporation au contact de la peau.

Le principe actif, quant à lui, sera choisi parmi des substances médicamenteuses solubles dans le solvant physiologiquement acceptable et capables de franchir de manière continue l'épiderme et le derme avec un flux suffisant pour donner une concentration sanguine thérapeutiquement efficace à partir d'une zone cutanée de dimension réduite mais suffisante.

De telles substances seront sélectionnées parmi les principes actifs qui montrent une demi-vie biologique relativement courte et un effet physiologique important à des taux plasmatiques faibles.

Outre l'estradiol, qui constitue un principe actif préféré selon l'invention, on peut citer différentes substances médicamenteuses qui pourront être incorporées avantageusement dans des compositions selon l'invention. Ces substances peuvent être choisies parmi les groupes suivants :
* un bronchodilatateur tel que le cromoglycate sodique, le salbutamol ou la théophylline
* un diurétique tel que le furosémide ou l'hydrochlorothiazide
* un agent antibactérien tel qu'une pénicilline, une céphalosporine, la tétracycline, l'oxytétracycline, la chlortétracycline ou le chloramphénicol
* un agent antiacnéique tel que l'érythromycine
* un sédatif ou tranquillisant tel que le pentobarbital ou son sel sodique, le secobarbital ou son sel sodique ou la codéine
* un psychostimulant tel que le 3-(2-aminopropyl)-indole acétate ou le 3-(2-aminobutyl)-indole acétate
* un anxiolytique tel que le diazépam, le chlordiazepoxide, la réserpine, la chlorpromazine ou le thiopropazate
* une hormone telle qu'un adrénocorticostéroïde par exemple la 6-méthylprednisolone
* un stéroïde androgénique par exemple la testostérone ou la méthyltestostérone
* un stéroïde oestrogénique par exemple l'estrone ou l'éthinyl estradiol
* un stéroïde progestatif par exemple la progesterone, la 17-α-hydroxyprogesterone, la medroxyprogesterone ou son acétate, la 19-norprogesterone, la norethindrone, la norethindrone acétate, la demegestrone ou le nomegestrol acétate
* une hormone thyroïdienne telle que la thyroxine
* un antipyrétique tel que l'acide acétylsalicylique, le salicylamide, le salicylate sodique ou le salicylate de méthyle
* un analgésique narcotique telle que la morphine ou un analgésique majeur
* un hypoglycémiant par exemple une sulfonyl-urée telle que le glypizide, le glyburique, le chlorpropamide ou l'insuline
* un antispasmodique tel que l'atropine ou le bromure de méthscopolamine
* un antitabagique tel que la lobéline ou la nicotine
* un antimalarique tel qu'une 4-aminoquinoléine ou une 9-aminoquinoléine
* un beta-bloquant tel que le metoprotol
* un agent antiarthritique tel que le sulindac
* un agent anti-inflammatoire non stéroïdien tel que l'ibuprofène ou le naproxène
* un agent antiostéoporotique tel que l'étidronate, le tiludronate ou leurs sels sodiques
* un agent de blanchiment cutané tel que l'acide ascorbique
* un vasodilatateur tel que le dipyridamole, la trinitrine ou le dinitrate d'isosorbide
* un antihypertenseur tel que le propranolol, la prazosine, le diltiazem ou la clonidine
* un antiparkinsonien tel que la méthyldopa ou la sélégiline
* un antimigraineux tel que la dihydroergotamine
* un antiulcéreux tel que la cimétidine
* un anticancéreux tel que le tamoxifène
* un apport nutritionnel tel que vitamines, acides aminés essentiels ou acides gras essentiels.

Ces principes actifs médicamenteux, comprenant l'estradiol, seront incorporés dans les compositions de l'invention à raison, notamment de 0,1% à 20% du poids de ces compositions étant entendu que chaque principe actif sera introduit à des concentrations individualisées et connues de l'état de la technique pour une administration transdermique ou adaptées à cette voie d'administration.

Par exemple, l'estradiol peut figurer dans les compositions de l'invention à raison de 0,5% à 6% du poids de cette composition notamment de 0,5% à 4%, de préférence de 1% à 2%.

Comme mentionné ci-dessus, les compositions de l'invention peuvent éventuellement contenir un principe actif formé d'une association de plusieurs substances médicamenteuses sélectionnées parmi les groupes énumérés précédemment.

A titre d'exemple, on peut citer une association estro-progestative pour le traitement de symptômes de la ménopause constituée d'un stéroïde oestrogénique tel que l'estradiol et d'un stéroïde progestatif tel que l'acétate de noréthindrone ou encore, une association contraceptive telle que levonorgestrel / estradiol.

De manière à atteindre une concentration sanguine efficace en principe actif sans pour autant recouvrir une surface de peau trop importante, on associe à la matrice polymérique et au principe actif, un promoteur d'absorption transcutanée. Ce dernier entre dans les compositions de l'invention avantageusement à raison de 15% à 30% du poids de cette composition, de préférence de 15% à 25% par exemple 20%.

Ce promoteur d'absorption est choisi de telle manière qu'il puisse amener des flux transdermiques importants pour atteindre les concentrations plasmatiques souhaitées moyennant un recouvrement cutané acceptable, c'est-à-dire inférieur à 150 cm² mais de préférence compris entre 10 et 40 cm² par exemple 30 cm².

Le promoteur d'absorption transcutané en question, pour être efficace, doit être capable de désorganiser de façon transitoire la barrière cutanée de manière à augmenter la perméabilité de la peau sans l'irriter tout en favorisant la diffusion du principe actif choisi selon une cinétique et une concentration suffisantes pouvant être maintenues pendant un certain temps.

Ce promoteur d'absorption transcutané sera sélectionné parmi des substances solubles dans le solvant physiologique non aqueux capable de s'évaporer rapidement au contact de la peau.

Des promoteurs d'absorption sont sélectionnés parmi les esters d'acides gras aliphatiques et alcools gras aliphatiques mentionnés précédemment sont rapportés ci-dessous, à savoir :
a) des esters d'acides gras aliphatiques de formule générale : 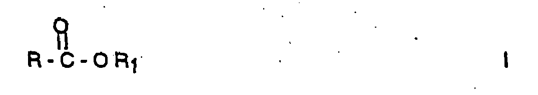 dans laquelle R représente un groupement alkyle ou alcényle, linéaire ou ramifié, en C₂-C₁₇ éventuellement substitué par un groupement hydroxyle, carboxylique, ou acyloxy en C₁-C₄ et R₁ représente un groupement alkyle, linéaire ou ramifié, en C₃-C₈ éventuellement substitué par un ou deux groupements hydroxyles tel que par exemple un groupement isopropyle, 2-éthyl-hexyle, 1,2-dihydroxy-éthyle ou R₁ représente un groupement -CH₂-CH₂-O-(CH₂)₂-O-CH₂-CH₃, l'ester d'acide gras aliphatique comportant un minimum de 10 atomes de carbone et un maximum de 2 groupements hydroxyles
b) des alcools gras aliphatiques de formule générale

   R₂ - OH II

   dans laquelle R₂ représente un groupement alkyle en C₁₀-C₂₀.

A titre de composés particuliers ayant montré les meilleures potentialités pour promouvoir l'absorption transcutanée de principes actifs, notamment l'estradiol, on peut citer :
le 2-éthyl-hexanoate de 2-éthyl-hexyle (Composé 1)
le myristate d'isopropyle (Composé 2)
le myristate de l'éther monoéthylique du diéthylèneglycol (Composé 3)
le palmitate d'isopropyle (Composé 4)
le 2-octyldodécanol (Composé 5)
l'undécylénate de 2-éthyl-hexyle (Composé 6)
le succinate de 2-éthyl-hexyle (Composé 7)
le 12-hydroxy-stéarate de 2-éthyl-hexyle (Composé 8)
le 12-acétoxy-stéarate de 2-éthyl-hexyle (Composé 9)
l'isostéarate de glycérol (Composé 10)
le laurate d'hexyle (Composé 11)

Le 2-éthyl-hexanoate de 2-éthyl-hexyle représente le promoteur d'absorption préféré notamment pour des compositions transdermiques selon l'invention dont le principe actif est l'estradiol.

Quant au solvant non aqueux physiologiquement acceptable, capable de dissoudre la matrice de relargage, le principe actif et le promoteur d'absorption transcutané, on le choisira parmi des composés de point d'ébullition relativement faible, à savoir inférieur à 100°C à pression atmosphérique, de façon qu'il puisse s'éliminer rapidement par évaporation au contact de la peau et aider de la même manière à la formation d'un film par séchage sans toutefois provoquer d'irritation locale.

De tels solvants physiologiquement acceptables sont généralement utilisés à raison de 44% à 84,9% du poids de la composition finale et peuvent être sélectionnés parmi des composés volatils tels que le dichlorométhane, l'éthanol, l'isopropanol ou l'acétate d'éthyle.

L'éthanol et l'isopropanol constituent des solvants de choix. Toutefois, l'éthanol représente un solvant préféré selon l'invention puisqu'il contribue avec efficacité à la formation de films particulièrement homogènes tout en s'évaporant rapidement au contact de la peau.

En conséquence, selon un de ses aspects particuliers, l'invention concerne une composition transdermique consistant en :
1) au plus 6% d'une matrice polymerique de relargage capable de former un film souple après séchage, choisie en particulier parmi des polymères ou copolymères cellulosiques tel que l'éthylcellulose
2) de 0,1 % à 20% d'un principe actif, en particulier de 1 % à 2% d'estradiol
3) de 15% à 30% d'un promoteur d'absorption transcutanée du principe actif, en particulier de 15% à 25% d'un ester d'acide gras ou d'un alcool gras choisi parmi :
   le 2-éthyl-hexanoate de 2-éthyl-hexyle
   le myristate d'isopropyle
   le myristate de l'éther monoéthylique du diéthylèneglycol
   le palmitate d'isopropyle
   le 2-octyldodécanol
   l'undécylénate de 2-éthyl-hexyle
   le succinate de 2-éthyl-hexyle
   le 12-hydroxystéarate de 2-éthyl-hexyle
   le 12-acétoxystéarate de 2-éthyl-hexyle
   l'isostéarate de glycérol
   le laurate d'hexyle
4) de 44% à 84,9% d'un solvant non aqueux physiologiquement acceptable capable de dissoudre la matrice de relargage, le principe actif et le promoteur d'absorption transcutanée ainsi que de s'éliminer rapidement par évaporation au contact de la peau, en particulier l'éthanol ou l'isopropanol.

Les compositions selon l'invention pour administration transdermique peuvent être préparées, de manière classique, en mélangeant les divers constituants dans les proportions choisies.

Par exemple, on peut, sous agitation, dissoudre le promoteur d'absorption transcutané dans le solvant physiologique puis ajouter le principe actif et finalement la matrice de relargage.

L'ensemble des substances entrant dans les compositions de l'invention constituent des produits connus ou pouvant être préparés par des méthodes connues, certains de ces produits étant disponibles dans le commerce.

Les compositions transdermiques de l'invention ainsi obtenues peuvent être appliquées par tout moyen sur une zone cutanée prédéterminée par exemple sur une zone comprise entre 10 et 40 cm² par exemple 30 cm² notamment et de préférence par pulvérisation directe au moyen d'une pompe doseuse de type connu et commercialisé sans l'aide d'agent propulseur tel qu'un gaz comprimé ou liquéfié.

Bien que l'état de la technique affirme l'inverse, on a remarqué, de manière surprenante, qu'une matrice de relargage formée d'éthylcellulose ne provoque pas d'obstruction par collage à la sortie de l'embout de la tête pulvérisatrice, de sorte que les compositions de l'invention pourront être pulvérisées sans nécessité de gaz propulseur et sans crainte de détérioration du récipient pulvérisateur.

Si on le désire, on peut néanmoins administrer les compositions de l'invention par pulvérisation à partir d'un récipient muni d'une valve doseuse, contenant en surplus un gaz propulseur comprimé tel que l'azote ou le protoxyde d'azote, ou liquéfié tel que le butane.

Selon un autre objet, l'invention concerne une matrice destinée à des compositions pharmaceutiques pour administration transdermique consistant en :
a) une matrice polymérique, pour le relargage d'un principe actif, capable de former un film souple après séchage.
b) un promoteur d'absorption transcutanée d'un principe actif
c) un solvant non aqueux physiologiquement acceptable capable de dissoudre la matrice de relargage et le promoteur d'absorption transcutanée ainsi que de s'éliminer rapidement par évaporation au contact de la peau.

La matrice polymérique sera sélectionnée parmi des substances polymériques ou copolymériques, c'est à dire parmi des polymères ou copolymères cellulosiques tels qu'explicités précédemment tandis que le promoteur d'absorption transcutanée figurera parmi des esters d'acide gras aliphatiques ou des alcools gras aliphatiques tels que décrits précédemment c'est à dire des esters de formule I ou alcools de formule II.

Quant au solvant non aqueux physiologiquement acceptable, il s'agit d'un composé de point d'ébullition inférieur à 100°C à pression atmosphérique tel que mentionné précédemment.

Ces différents composants de la matrice pour composition pharmaceutique transdermique seront répartis de telle manière qu'au sein de la composition pharmaceutique en question contenant le principe actif, la matrice de relargage représente de 0% à 6%, le promoteur d'absorption transcutanée représente de 15% à 30% et le solvant non aqueux physiologiquement acceptable représente de 44% à 84,9%, ces pourcentages étant exprimés en poids de la composition pharmaceutique finale.

Ces matrices pour compositions transdermiques selon l'invention peuvent être préparées, de manière classique, en mélangeant, dans les proportions choisies, les divers ingrédients les constituant.

Les compositions filmogènes de l'invention ainsi que les matrices pour compositions transdermiques selon l'invention présentent des avantages incontestables puisqu'elles sont capables de provoquer la diffusion transcutanée d'un principe actif, par exemple l'estradiol, de manière à produire des taux plasmatiques constants et contrôlés sur une période prolongée d'au moins 12 heures à partir d'une zone de recouvrement cutanée de l'ordre de 10 à 40 cm².

Les taux sanguins en principe actif ainsi fournis sont compatibles avec un traitement thérapeutique contrairement aux taux libérés par les compositions et matrices pour compositions transdermiques connues telles que celles décrites par exemple dans le brevet EP 0319555.

En outre, les compositions et matrices pour compositions transdermiques selon l'invention, tout en étant dépourvues de toute odeur désagréable, s'étalent en un film homogène sur la zone cutanée sélectionnée et, à cette fin, ne nécessite pas forcément l'intermédiaire d'agents gazeux propulseurs nocifs pour l'environnement.

Ces films sont suffisamment souples et résistants à l'abrasion pour éviter toute détérioration sur la peau d'un patient et présentent une meilleure tolérance que les dispositifs transdermiques connus puisqu'en raison de leur minceur et de l'absence de tout recouvrement les échanges gazeux et aqueux avec l'extérieur ne sont pas forcément perturbés.

Enfin, les compositions de l'invention, sous forme de film souple, procurent un meilleur confort pour le patient qu'un patch transdermique et, grâce à leur transparence, sont totalement invisibles.

Divers essais ont été effectués ***in vitro*** comme ***in vivo*** de manière à mettre en évidence les caractéristiques et particularités des compositions de l'invention.

### 1. Essais in vitro

### A. Matrice : 5% d'éthylcellulose

### Principe actif : estradiol

Le passage transcutané d'un principe actif incorporé dans un véhicule promoteur d'absorption peut être estimé quantitativement par la mesure du flux de ce principe actif susceptible de traverser la peau.

Les essais, impliquant des compositions de l'invention, ont été réalisés ***in vitro*** en cellules de diffusion de type Frantz qui permettent d'obtenir des conditions expérimentales très reproductibles facilitant les études comparatives.

Ces cellules de diffusion, qui possèdent un compartiment récepteur d'un volume de 30 ml, sont spécialement élaborées dans le but de pouvoir tester des formulations de type "spray" à pulvériser sur une surface cutanée de 10 cm².

Dans le test pratiqué, selon la méthode préconisée dans Curr. Probl. Dermatol. 7, 58-68 (1978), on a donc étudié l'absorption percutanée d'estradiol traversant des biopsies de 10 cm² de peau dorsale de rat sans poils placées sur des cellules de diffusion en question.

A cet effet, on a appliqué, par pulvérisation, 50 µl d'une composition de l'invention contenant l'estradiol et on a dosé, après 8, 24 et 30 heures, ce principe actif dans le liquide récepteur en contact avec la face dermique de la peau.

La perméabilité de la peau au passage du principe actif étant parfois très différente d'un lot à l'autre d'animaux, les résultats obtenus sont essentiellement comparatifs à l'intérieur d'une même série d'études.

### a) Composition à 2% d'estradiol

| | % en poids |
|---|---|
| Ethylcellulose | 5% |
| Estradiol | 2% |
| Promoteur d'absorption transcutanée | 20% |
| Ethanol | 73% |

Dans une première série d'essais, effectués avec les Composés 1, 2, 5, 6, 8, 9 et 11, on a enregistré des flux d'estradiol variant de 0,115 à 0,330 µg. h⁻¹. cm⁻², dans une deuxième série avec les Composés 1, 2, 7 et 10, des flux allant de 0,121 à 0,290 µg. h⁻¹. cm⁻² et dans une troisième série avec les Composés 1, 3 et 4, des flux compris entre 0,159 et 0,280 µg. h⁻¹. cm⁻².

Ces résultats montrent que les flux engendrés par les Composés 2 à 11 sont comparables à ceux enregistrés avec le Composé 1 sans qu'aucune différence réellement significative n'ait été enregistrée.

Une série d'essais supplémentaires a été pratiquée avec une composition de l'invention de formulation suivante :

| | % en poids |
|---|---|
| Ethylcellulose | 5% |
| Estradiol | 2% |
| Composé 1 | W% |
| Ethanol | (93-W) % |

On a enregistré les résultats rapportés ci-dessous :

| **Composé 1 (W %)** | **Flux (µg. h⁻¹. cm⁻²)** |
|---|---|
| 0 | 0,198 ± 0,038 |
| 5 | 0,310 ± 0,032 |
| 10 | 0,460 ± 0,066 |
| 15 | 0,501 ± 0,122 |
| 20 | 0,603 ± 0,136 |

Ces résultats montrent que les flux de diffusion de l'estradiol augmentent proportionnellement à la concentration du promoteur au sein de la composition.

Toutefois, au delà de 20%, on n'enregistre plus d'augmentation caractéristique des flux transcutanés d'estradiol, ceux-ci restant néanmoins élevés comme le prouvent les résultats ci-dessous:

| **Composé 1 (W%)** | **Flux (µg. h⁻¹. cm⁻²)** |
|---|---|
| 20 | 0,369 ± 0,058 |
| 30 | 0,197 ± 0,019 |

A titre comparatif, on à également effectué des tests à partir :
- soit d'une composition selon l'invention comprenant, en poids :

| | |
|---|---|
| Ethylcellulose | 5% |
| Estradiol | 2% |
| Composé 1 | 20% |
| Ethanol | 73% |

- soit de compositions de l'état de la technique représentée par le brevet EP 0319555 (Compositions X et Y) comprenant, en poids :

| | |
|---|---|
| Estradiol | 2% |
| n-Butyl ester de l'acide polyméthacrylique | 3,66% |
| Polyvinylpyrrolidone VA (PVP VA*) (solution éthanolique à 50%) | 6,66% |
| Ethanol | 11,66% |
| Sorbitan macrogollaurate | 1,66% |
| Chlorure de méthylène | 74,36% |

| | |
|---|---|
| * PVP VA : deux types de PVP VA ont été utilisés l'un à 30% de polyvinylpyrrolidone (PVP VA 335 ISP) dans la Composition X et l'autre à 70% de polyvinylpyrrolidone (PVP VA 735 ISP) dans la Composition Y | |

Les résultats obtenus ont été les suivants :

| **Compositions** | **Flux (µg. h⁻¹. cm⁻²)** |
|---|---|
| De l'invention | 0,295 ± 0,105 |
| Composition X | 0,032 ± 0,01 |
| Composition Y | 0,024 ± 0,007 |

Ces résultats montrent la nette supériorité des compositions de l'invention sur les compositions antérieures, les flux enregistrés étant 8 à 9 fois plus importants.

Un test supplémentaire, entrepris avec les Compositions X et Y pulvérisées sur 10 cm² d'aluminium puis mises en contact avec la peau après évaporation des solvants selon la technique décrite dans le brevet EP 0319555, cité ci-dessus, a mis en évidence des flux de diffusion transcutanée d'estradiol de 0,009 ± 0,011 et 0,002 ± 0,003 µg.h⁻¹.cm⁻² respectivement.

### b) Compositions à 1% d'estradiol

Dans une autre série d'essais, on a pratiqué des tests analogues en vue de mettre en évidence la supériorité des compositions de l'invention sur des compositions transdermiques identiques dont on a remplacé le promoteur d'absorption transcutanée de manière à estimer les qualités des composés de formule I.

A cet effet, on a utilisé des compositions répondant à la formulation suivante:

| | |
|---|---|
| Ethylcellulose | 5% en poids |
| Estradiol | 1% en poids |
| Composé Z | 20% en poids |
| Ethanol | 74% en poids |

le Composé Z étant soit un promoteur d'absorption transcutanée de formule I ci-dessus, soit un composé issu de l'état de la technique.

De même, on a pratiqué un test comparatif à partir d'un gel à l'estradiol (estradiol : 0,06%*; éthanol 95° ; 40%*; CARBOPOL® : 1%*; triéthanolamine : 1%*; eau purifiée : q.s.p. 100%) commercialisé sous la marque OESTROGEL®.
* % en poids

On a obtenu les résultats suivants :
1) Compositions pulvérisables

| **Composé Z** | **Flux (µg. h⁻¹. cm⁻²)** |
|---|---|
| Composé 1 | 0,116 ± 0,042 |
| Ether monoéthylique | |
| du diéthylèneglycol | |
| (TRANSCUTOL® ) | 0,032 ± 0,014 |
| Diméthylisosorbide | 0,028 ± 0,021 |

2) Gel

| | |
|---|---|
| OESTROGEL® | 0,014 ± 0,003 |

Ces résultats montrent que les flux de diffusion transcutanée d'estradiol par cm², générés par la composition de l'invention contenant le Composé 1, sont de loin supérieurs à ceux obtenus à partir de compositions pulvérisables dont on a remplacé ce Composé 1 puisque de 3 à 4 fois plus importants.

Dans le cas du gel, on observe que les flux de diffusion transcutanée du principe actif sont 7 à 8 fois plus faibles que ceux produits par la composition de l'invention.

Exprimés en surface réelle d'application, à savoir 30 cm² pour la composition de l'invention et 100 cm² pour le produit OESTROGEL® , ces résultats traduisent qu'une quantité deux fois plus importante de principe actif diffuse en 24 heures à partir de la composition de l'invention par rapport au gel puisque l'on a enregistré environ 40 µg pour le gel contre 80 µg pour la composition de l'invention.

D'autre part, des études expérimentales pratiquées avec des compositions filmogènes à 1% d'estradiol selon l'invention, lavées 8 heures après application, n'ont révélé aucune incidence sur la diffusion du principe actif même 30 heures après son application sur la peau.

De l'ensemble des résultats rapportés précédemment, on peut conclure que les compositions transdermiques de l'invention présentent une nette supériorité sur les autres compositions testées due notamment à la présence d'un promoteur d'absorption transcutanée du principe actif, capable de contribuer efficacement à la production de flux importants de diffusion transdermique de ce principe actif.

Ces flux transdermiques importants laissent présager une meilleure performance des compositions de l'invention par exemple pour générer, à partir d'une même zone d'application cutanée, des taux plasmatiques en principe médicamenteux mieux adaptés à la thérapeutique.

### c) Compositions à 2%, 4% ou 6% d'estradiol

On a pratiqué des tests analogues à celui décrit au paragraphe I. A. ci-dessus en vue de montrer l'influence de la concentration en estradiol sur les flux transcutanés.

A cet effet, on a utilisé des compositions répondant à la formulation suivante :

| | % en poids |
|---|---|
| Ethylcellulose | 5% |
| Estradiol | Es% |
| Composé 1 | 20% |
| Solvant | (75-Es)% |

On a obtenu les résultats suivants :

| **Estradiol (Es%)** | **Solvant : (75-Es)%** | **Flux (µg. h⁻¹. cm⁻²)** |
|---|---|---|
| 2 | Ethanol: 73% | 0,369 ± 0,058 |
| 4 | Ethanol / Isopropanol 30/70:71% | 0,509 ± 0,050 |
| 6 | Isopropanol : 69% | 0,769 ± 0,159 |

### B. Matrice : 0%, 2%, 3%, 4%, 5% ou 6% d'éthylcellulose

### Principe actif : estradiol

Des tests analogues à celui décrit au paragraphe I. A. ont été réalisés in vitro en cellules, de diffusion de type Frantz à partir d'une composition de l'invention de formulation suivante :

| | % en poids |
|---|---|
| Ethylcellulose | Et% |
| Estradiol | 2% |
| Composé 1 | 20% |
| Ethanol | (78-Et)% |

On a enregistré les résultats rapportés ci-dessous :

| **Ethylcellulose (Et%)** | **Flux (µg. h⁻¹. cm⁻²)** |
|---|---|
| 0 | 0,214 ± 0,032 |
| 2 | 0,362 ± 0,079 |
| 3 | 0,445 ± 0,099 |
| 4 | 0,354 ± 0,092 |
| 5 | 0,347 ± 0,095 |
| 6 | 0,397 ± 0,034 |

Ces résultats montrent que la concentration en éthylcellulose n'apporte que peu d'influence sur les flux d'estradiol à l'équilibre.

### C. Matrice : 5% d'éthylcellulose

### Principes actifs : estradiol, sélégiline, ibuprofène, donidine, testostérone, acétate de norethindrone, acide acétylsalicylique

On a utilisé à cet effet un test in vitro en cellules de diffusion de type Frantz analogue à celui décrit précédemment pour l'estradiol (paragraphe I. A. ci-dessus).

L'ensemble des études quantitatives pratiquées a porté sur les principes actifs radiomarqués.

A cet effet, on a appliqué par pulvérisation 50µl d'une composition contenant 10µ Ci de principe actif radiomarqué sur 10cm² de peau et on a dosé après 7, 24 et 30 heures la radioactivité totale par scintillation liquide dans des prélèvements de 1ml de liquide récepteur en présence d'un mélange scintillant.

Les essais avec différents principes actifs ont été réalisés comparativement à des compositions renfermant le 17β estracliol radiomarqué au tritium par dilution isotopique (2, 4, 6, 7 - ³H - estradiol)

### a) Principe actif : sélégiline

Les essais, réalisés sur la molécule marquée au tritium par dilution isotopique, ont fournis les résultats suivants :

| 1ère série d'essais | | |
|---|---|---|
| | % en poids | Flux (µg. h⁻¹. cm⁻²) |
| Ethylcellulose | 5 | |
| Estradiol | 2 | 0,732 ± 0,110 |
| Composé 1 | 20 | |
| Ethanol | 73 | |
| | | |
| Ethylcellulose | 5 | |
| Sélégiline | 4 | 4,010 ± 0,898 |
| Composé 7 | 20 | |
| Ethanol | 71 | |

| 2ème série d'essais | | |
|---|---|---|
| | % en poids | Flux (µg. h⁻¹. cm⁻²) |
| Ethylcellulose | 5 | |
| Estradiol | 2 | 0,598 ± 0,173 |
| Composé 1 | 20 | |
| Ethanol | 73 | |
| Ethylcellulose | 5 | |
| Sélégiline | 10 | 7,878 ± 2,600 |
| Composé 1 | 20 | |
| Ethanol | 65 | |
| Ethylcellulose | 5 | |
| Sélégiline | 10 | 6,645 ± 0,809 |
| Composé 2 | 20 | |
| | 65 | |
| Ethanol | | |

### b) Principe actif : ibuprofène

Les essais effectués sur la molécule radiomarquée au tritium par dilution isotopique, ont fourni les résultats suivants :

| 1ère série d'essais | | |
|---|---|---|
| | % en poids | Flux (µg.h⁻¹. cm⁻²) |
| Ethylcellulose | 5 | |
| Estradiol | 2 | 0,909 ± 0,184 |
| Composé 1 | 20 | |
| Ethanol | 73 | |
| | | |
| Ethylcellulose | 5 | |
| Ibuprofène | 2 | 1,432 ± 0,307 |
| Composé 1 | 20 | |
| Ethanol | 73 | |

Un essai comparatif supplémentaire effectué avec une composition contenant 5% d'éthylcellulose, 2% d'ibuprofène et 93% d'éthanol a fourni des flux de diffusion transcutanés de 0,780 µg. h⁻¹. cm⁻².

Ce résultat montre nettement que le Composé 1 joue un rôle de promoteur d'absorption transdermique pour l'ibuprofène.

| 2ème série d'essais | | |
|---|---|---|
| | % en poids | Flux (µg. h⁻¹. cm⁻²) |
| Ethylcellulose | 5 | |
| Estradiol | 2 | 0,805 ± 0,102 |
| Composé 1 | 20 | |
| Ethanol | 73 | |
| | | |
| Ethylcellulose | 5 | |
| Ibuprofène | 2,5 | 1,911 ± 0,137 |
| Composé 1 | 20 | |
| Ethanol | 72,5 | |
| | | |
| Ethylcellulose | 5 | |
| Ibuprofène | 2 | 1,272 ± 0,292 |
| Composé 7 | 20 | |
| Ethanol | 73 | |

### c) Principe actif : clonidine

Les essais, réalisés avec la molécule marquée au tritium par dilution isotopique (chlorhydrate de phényl-4-³H-clonidine), ont fourni les résultats suivants :

| | % en poids | Flux (µg. h⁻¹. cm⁻²) |
|---|---|---|
| Ethylcellulose | 5 | |
| Estradiol | 2 | 0,761 ± 0,134 |
| Composé 1 | 20 | |
| Ethanol | 73 | |
| | | |
| Ethylcellulose | 5 | |
| Clonidine | 2 | 0,213 ± 0,127 |
| Composé 1 | 20 | |
| Ethanol | 73 | |

Un essai comparatif supplémentaire effectué avec une composition contenant 5% d'éthylcellulose, 2% de clonidine et 93% d'éthanol a fourni des flux de diffusion transcutanés de 0,079 ± 0,118 µg. h⁻¹. cm⁻².

Ce résultat montre que le Composé 1 joue un rôle de promoteur d'absorption transdermique pour la clonidine.

### d) Principe actif : testostérone

Les essais, réalisés sur la molécule marquée au tritium par dilution isotopique (1,2, 6, 7-³H-testostérone), ont fourni les résultats suivants :

| 1ère série d'essais | | |
|---|---|---|
| | % en poids | Flux (µg. h⁻¹. cm⁻²) |
| Ethylcellulose | 5 | |
| Estradiol | 2 | 0,679 ± 0,065 |
| Composé 1 | 20 | |
| Ethanol | 73 | |
| | | |
| Ethylcellulose | 5 | |
| Testostérone | 2 | 1,637 ± 0,164 |
| Composé 1 | 20 | |
| Ethanol | 73 | |
| | | |
| Ethylcellulose | 5 | |
| Testostérone | 2 | 1,274 ± 0,128 |
| Composé 1 | 15 | |
| Ethanol | 78 | |

| 2ème série d'essais | | |
|---|---|---|
| | % en poids | Flux (µg. h⁻¹. cm⁻²) |
| Ethylcellulose | 5 | |
| Testostérone | 2 | 1,419 ± 0,189 |
| Composé 1 | 20 | |
| Ethanol | 73 | |

Un essai supplémentaire effectué avec une composition contenant 5% d'éthylcellulose, 2% de testostérone et 93% d'éthanol a fourni des flux de diffusion transcutanés de 0,443 ± 0,190 µg. h⁻¹. cm⁻².

Ce résultat montre que le Composé 1 a bien un rôle de promoteur d'absorption transdermique pour la testostérone.

### e) Principe actif : acétate de noréthindrone

Les essais, réalisés avec la molécule marquée au tritium par dilution isotopique, ont fourni les résultats suivants

| 1ère série d'essais | | |
|---|---|---|
| | % en poids | Flux (µg.h⁻¹.cm⁻²) |
| Ethylcellulose | 5 | |
| Estradiol | 2 | 0,588 ± 0,077 |
| Composé 1 | 20 | |
| Ethanol | 73 | |
| | | |
| Ethylcellulose | 5 | |
| Acétate de noréthindrone | 3 | 0,438 ± 0,194 |
| Composé 1 | 20 | |
| Ethanol | 72 | |
| | | |
| Ethylcellulose | 5 | |
| Acétate de noréthindrone | 2 | |
| Composé 7 | 20 | 0,241 ± 0,101 |
| Ethanol | 73 | |

| 2ème série d'essais | | |
|---|---|---|
| | % en poids | Flux (µg. h⁻¹. cm⁻²) |
| Ethylcellulose | 5 | |
| Estradiol | 2 | 0,276 ± 0,143 |
| Composé 1 | 20 | |
| Ethanol | 73 | |
| | | |
| Ethylcellulose | 5 | |
| Acétate de noréthindrone | 2 | 0,341 ± 0,078 |
| Composé 1 | 20 | |
| Ethanol | 73 | |

Un essai supplémentaire effectué avec une composition contenant 5% d'éthylcellulose, 2% d'acétate de noréthindrone et 93% d'éthanol a fourni des flux de diffusion transcutanés de 0,066 t 0,026 µg. h⁻¹. cm⁻², ce qui montre que le Composé 1 a bien un rôle de promoteur d'absorption transdermique pour l'acétate de noréthindrone.

### f) Principe actif : acide acétylsalicylique

Les essais, réalisés avec la molécule marquée au ¹⁴C par dilution isotopique (acide carboxyl-¹⁴C acétylsalicylique), ont fourni les résultats suivants :

| | % en poids | Flux (µg. h⁻¹. cm⁻²) |
|---|---|---|
| Ethylcellulose | 5 | |
| Estradiol | 2 | 0,554 ± 0,108 |
| Composé 1 | 20 | |
| Ethanol | 73 | |
| | | |
| Ethylcellulose | 5 | |
| Acide acétylsalicylique | 2 | 1,724 ± 0,153 |
| Composé 1 | 20 | |
| Ethanol | 73 | |
| | | |
| Ethylcellulose | 5 | |
| Acide acétylsalicylique | 2 | 1,689 ± 0,127 |
| Composé 7 | 20 | |
| Ethanol | 73 | |

### D. Matrice : polyvinylpyrrolidone / acétate de vinyle

### Principe actif : estradiol

On a pratiqué une série d'essais supplémentaires analogues à celui décrit au paragraphe I. A. de manière à déterminer les flux de diffusion transcutanés d'estradiol à partir de compositions de l'invention comportant une matrice de polyvinylpyrrolidone / acétate de vinyle (PVP VA).

A cet effet, on a utilisé des compositions de formulation suivante :

| | % en poids |
|---|---|
| PVP VA | P% |
| Estradiol | 2% |
| Composé 1 | 20% |
| Ethanol | (78-P)% |

comparativement à une Composition C de l'invention comportant une matrice formée d'éthylcellulose :

| | % en poids |
|---|---|
| Ethylcellulose | 5% |
| Estradiol | 2% |
| Composé 1 | 20% |
| Ethanol | 73% |

On a obtenu les résultats suivants :

| **PVP VA (P%)** | **Flux (µg. h⁻¹. cm⁻²)** |
|---|---|
| 2 | 0,165 ± 0,014 |
| 3 | 0,212 ± 0,026 |
| 4 | 0,209 ± 0,023 |
| 5 | 0,205 ± 0,037 |
| Composition C | 0,321 ± 0,073 |

En vue d'un test comparatif, on a tenté de préparer une composition transdermique comportant 2% d'estradiol, une matrice, un solvant et un gaz propulseur analogues à ceux décrits dans le brevet EP 0319555 à savoir 2,5% de PVP VA, 2,5% de n-butyl ester de l'acide méthacrylique, 15% d'éthanol, 13% de dichlorométhane et 61,5% de fréon auxquels on a ajouté 5% de Composé 1, promoteur utilisé dans la présente invention.

Toutefois, cette composition à 2% d'estradiol n'a pu être réalisée, ce principe actif ne parvenant pas à se dissoudre dans un tel mélange.

Enfin, une composition de formulation :

| | % en poids |
|---|---|
| PVP VA | 3% |
| Estradiol | 2% |
| Composé 1 | 25% |
| Ethanol | 70% |

a fourni des flux de diffusion transcutanés de 0,232 ± 0,028 µg. h⁻¹. cm⁻².

### II. Essais in vivo

Des essais ont également été effectués ***in vivo*** sur le microporc de type Yucatan pesant environ 13 kgs sur lequel on applique :
- soit 100 µl d'une composition à 2% d'estradiol selon l'invention, par pulvérisation sur 30 cm² de peau, ce qui équivaut à 1,5 mg d'estradiol
- soit 2,5 g de gel OESTROGEL® par étalement sur 100 cm² de peau, ce qui équivaut également à 1,5 mg d'estradiol.

On a alors déterminé, à différents intervalles de temps, les taux plasmatiques en estradiol.

Dans ces conditions d'application, comparables à celles pouvant être utilisées chez la femme, les concentrations plasmatiques en estradiol produites par la composition de l'invention et le gel OESTROGEL® ont été respectivement d'environ 390 pg/ml et 170 pg/ml, 8 heures après application et environ 304 pg/ml et 160 pg/ml, 24 heures après application.

La composition de l'invention est, par conséquent, environ deux fois plus performante que le gel OESTROGEL® pour la production de taux sanguins en estradiol durant une période de 24 heures.

Des tests comparatifs analogues, pratiqués avec les Compositions X et Y selon l'état de la technique, ont également mis en évidence une supériorité significative des compositions de l'invention pour la production de taux sanguins importants et prolongés en estradiol.

Les Exemples, non limitatifs suivants, illustrent la préparation de compositions de l'invention ainsi que des matrices pour compositions transdermiques selon l'invention.

### EXEMPLE 1

### Composition transdermique contenant l'estradiol

On prépare 100 g d'une composition transdermique de formulation suivante :

| | % en poids |
|---|---|
| Ethylcellulose 6 mPa.sec | 5% |
| Estradiol | 2% |
| 2-Ethyl-hexanoate de 2-éthyl-hexyle | 20% |
| Ethanol | 73% |

en mélangeant pendant 30 secondes et sous agitation magnétique 73 g d'éthanol et 20 g de 2-éthyl-hexanoate de 2-éthyl-hexyle.

On ajoute alors, par petites fractions, 2 g d'estradiol au mélange obtenu puis, après dissolution complète (5 minutes), on introduit 5 g d'éthylcellulose 6 mPa.sec, sous vive agitation, afin d'éviter la formation de grumeaux. La solution finale obtenue est homogène légèrement opalescente.

En vue d'une administation par pulvérisation, on remplit des boîtiers en aluminium à l'aide de 5 ml de la solution précédemment obtenue et on les munit d'une vasopompe à sertir comportant un bouton poussoir.

On actionne deux fois la pompe pour l'amorcer avant sa première utilisation.

### EXEMPLES 2 à 38

### Compositions transdermiques contenant l'estradiol

En utilisant le même procédé que dans l'Exemple 1, on a préparé les compositions transdermiques de formulations suivantes :

| | | % en poids |
|---|---|---|
| **EX. 2** | Ethylcellulose | 5% |
| | Estradiol | 1% |
| | 2-Ethyl-hexanoate de 2-éthyl-hexyle | 20% |
| | Ethanol | 74% |
| **EX. 3** | Ethylcellulose | 5% |
| | Estradiol | 2% |
| | 12-Acétoxystéarate de 2-éthyl-hexyle | 20% |
| | Ethanol | 73% |
| **EX. 4** | Ethylcellulose | 5% |
| | Estradiol | 1,5% |
| | Laurate d'hexyle | 20% |
| | Ethanol | 73,5% |
| **EX. 5** | Ethylcellulose | 5% |
| | Estradiol | 2,5% |
| | 2-Octyl-dodécanol | 20% |
| | Ethanol | 72,5% |
| **EX. 6** | Ethylcellulose | 5% |
| | Estradiol | 3% |
| | 12-Hydroxystéarate de 2-éthyl-hexyle | 20% |
| | Ethanol | 72% |
| **EX. 7** | Ethylcellulose | 2% |
| | Estradiol | 1% |
| | 2-Ethyl-hexanoate de 2-éthyl-hexyle | 20% |
| | Ethanol | 77% |
| **EX. 8** | Ethylcellulose | 5% |
| | Testosterone | 2% |
| | 2-Ethyl-hexanoate de 2-éthyl-hexyle | 20% |
| | Ethanol | 73% |
| **EX. 9** | Ethylcellulose | 5% |
| | Estradiol | 2% |
| | 2-Ethyl-hexanoate de 2-éthyl-hexyle | 5% |
| | Ethanol | 88% |
| **EX. 10** | Ethylcellulose | 5% |
| | Estradiol | 2% |
| | 2-Ethyl-hexanoate de 2-éthyl-hexyle | 15% |
| | Ethanol | 78% |
| **EX. 11** | Ethylcellulose | 5% |
| | Estradiol | 2% |
| | 2-Ethyl-hexanoate de 2-éthyl-hexyle | 20% |
| | Isopropanol | 73% |
| **EX. 12** | Ethylcellulose | 5% |
| | Estradiol | 4% |
| | 2-Ethyl-hexanoate de 2-éthyl-hexyle | 20% |
| | Ethanol / isopropanol 30/70 | 71% |
| **EX. 13** | Ethylcellulose | 5% |
| | Estradiol | 6% |
| | 2-Ethyl-hexanoate de 2-éthyl-hexyle | 20% |
| | Isopropanol | 69% |
| **EX. 14** | Estradiol | 2% |
| | 2-Ethyl-hexanoate de 2-éthyl-hexyle | 20% |
| | Ethanol | 78% |
| **EX. 15** | Ethylcellulose | 2% |
| | Estradiol | 2% |
| | 2-Ethyl-hexanoate de 2-éthyl-hexyle | 20% |
| | Ethanol | 76% |
| **EX. 16** | Ethylcellulose | 3% |
| | Estradiol | 2% |
| | 2-Ethyl-hexanoate de 2-éthyl-hexyle | 20% |
| | Ethanol | 75% |
| **EX. 17** | Ethylcellulose | 4% |
| | Estradiol | 2% |
| | 2-Ethyl-hexanoate de 2-éthyl-hexyle | 20% |
| | Ethanol | 74% |
| **EX. 18** | Ethylcellulose | 6% |
| | Estradiol | 2% |
| | 2-Ethyl-hexanoate de 2-éthyl-hexyle | 20% |
| | Ethanol | 72% |
| **EX. 19** | Ethylcellulose | 5% |
| | Sélégiline | 10% |
| | 2-Ethyl-hexanoate de 2-éthyl-hexyle | 20% |
| | Ethanol | 65% |
| **EX. 20** | Ethylcellulose | 5% |
| | Sélégiline | 4% |
| | Succinate de 2-éthyl-hexyle | 20% |
| | Ethanol | 71% |
| **EX. 21** | Ethylcellulose | 5% |
| | Sélégiline | 10% |
| | Myristate d'isopropyle | 20% |
| | Ethanol | 65% |
| **EX. 22** | Ethylcellulose | 5% |
| | Ibuprofène | 2% |
| | 2-Ethyl-hexanoate de 2-éthyl-hexyle | 20% |
| | Ethanol | 73% |
| **EX. 23** | Ethylcellulose | 5% |
| | Ibuprofène | 2,5% |
| | 2-Ethyl-hexanoate de 2-éthyl-hexyle | 20% |
| | Ethanol | 72,5% |
| **EX. 24** | Ethylcellulose | 5% |
| | Ibuprofène | 2% |
| | Succinate de 2-éthyl-hexyle | 20% |
| | Ethanol | 73% |
| **EX. 25** | Ethylcellulose | 5% |
| | Clonidine | 2% |
| | 2-Ethyl-hexanoate de 2-éthyl-hexyle | 20% |
| | Ethanol | 73% |
| **EX. 26** | Ethylcellulose | 5% |
| | Testostérone | 2% |
| | 2-Ethyl-hexanoate de 2-éthyl-hexyle | 15% |
| | Ethanol | 78% |
| **EX. 27** | Ethylcellulose | 5% |
| | Acétate de noréthindrone | 3% |
| | 2-Ethyl-hexanoate de 2-éthyl-hexyle | 20% |
| | Ethanol | 72% |
| **EX. 28** | Ethylcellulose | 5% |
| | Acétate de noréthindrone | 2% |
| | Succinate de 2-éthyl-hexyle | 20% |
| | Ethanol | 73% |
| **EX. 29** | Ethylcellulose | 5% |
| | Acétate de noréthindrone | 2% |
| | 2-Ethyl-hexanoate de 2-éthyl-hexyle | 20% |
| | Ethanol | 73% |
| **EX. 30** | Ethylcellulose | 5% |
| | Acide acétylsalicylique | 2% |
| | 2-Ethyl-hexanoate de 2-éthyl-hexyle | 20% |
| | Ethanol | 73% |
| **EX. 31** | Ethylcellulose | 5% |
| | Acide acétylsalicylique | 2% |
| | Succinate de 2-éthyl-hexyle | 20% |
| | Ethanol | 73% |
| **EX. 32** | PVP VA | 2% |
| | Estradiol | 2% |
| | 2-Ethyl-hexanoate de 2-éthyl-hexyle | 20% |
| | Ethanol | 76% |
| **EX. 33** | PVP VA | 3% |
| | Estradiol | 2% |
| | 2-Ethyl-hexanoate de 2-éthyl-hexyle | 20% |
| | Ethanol | 75% |
| **EX. 34** | PVP VA | 4% |
| | Estradiol | 2% |
| | 2-Ethyl-hexanoate de 2-éthyl-hexyle | 20% |
| | Ethanol | 74% |
| **EX. 35** | PVP VA | 5% |
| | Estradiol | 2% |
| | 2-Ethyl-hexanoate de 2-éthyl-hexyle | 20% |
| | Ethanol | 73% |
| **EX. 36** | PVP VA | 3% |
| | Estradiol | 2% |
| | 2-Ethyl-hexanoate de 2-éthyl-hexyle | 25% |
| | Ethanol | 70% |
| **EX. 37** | Ethylcellulose | 5% |
| | Estradiol | 2% |
| | 2-Ethyl-hexanoate de 2-éthyl-hexyle | 30% |
| | Ethanol | 63% |

### EXEMPLE 38

### Matrice pour composition transdermique

On prépare 98 g d'une matrice pour composition transdermique en mélangeant, pendant 30 secondes, 73 g d'éthanol et 20 g de 2-éthyl-hexanoate de 2-éthyl-hexyle. On ajoute alors 5 g d'éthylcellulose 6 mPa.sec, sous vive agitation, afin d'éviter la formation de grumeaux.

La matrice, ainsi obtenue, est prête à recevoir un principe actif, par incorporation, de manière à former une composition pharmaceutique contenant 2% en poids de ce principe actif, applicable par pulvérisation.

## Revendications

1. Composition pharmaceutique pour administration transdermique **caractérisée en ce qu'**elle consiste en :
a) une matrice polymérique de relargage capable de former un film souple après séchage, choisie parmi des polymères ou copolymères cellulosiques ;
b) un principe actif ;
c) 15 à 30 %, par rapport au poids de la composition, d'un promoteur d'absorption transcutanée du principe actif ;
d) un solvant non aqueux, physiologiquement acceptable capable de dissoudre la matrice de relargage, le principe actif et le promoteur d'absorption transcutanée ainsi que de s'éliminer rapidement par évaporation au contact de la peau,
le promoteur d'absorption transcutanée étant choisi parmi :
. un ester d'acide gras aliphatique soluble dans le solvant non aqueux physiologiquement acceptable et de formule générale : 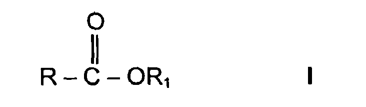 dans laquelle R représente un groupement alkyle ou alcényle, linéaire ou ramifié, en C₂-C₁₇ éventuellement substitué par un groupement hydroxyle, carboxylique ou acyloxy en C₁-C₄ et R₁ représente un groupement alkyle, linéaire ou ramifié, en C₃-C₈ éventuellement substitué par un ou deux groupements hydroxyles ou R₁ représente un groupement -CH₂-CH₂-O-(CH₂)₂-O-CH₂-CH₃, l'ester d'acide gras aliphatique comportant un minimum de 10 atomes de carbone et un maximum de 2 groupements hydroxyles
. un alcool gras aliphatique, soluble dans le solvant non aqueux physiologiquement acceptable de formule générale :
R₂-OH II
dans laquelle R₂ représente un groupement alkyle en C₁₀-C₂₀.

2. Composition pharmaceutique selon la Revendication 1 **caractérisée en ce que** la matrice polymérique de relargage représente au plus 6 % du poids de la composition.

3. Composition pharmaceutique selon la Revendication 2 **caractérisée en ce que** la matrice polymérique de relargage est présente à raison de 1 % à 5 % du poids de la composition.

4. Composition pharmaceutique selon une des Revendications 1 à 3
**caractérisée en ce que** le principe actif est présent à raison de 0, 1 % à 20 % du poids de la composition.

5. Composition pharmaceutique selon une des Revendications 1 à 4
**caractérisée en ce que** le promoteur d'absorption transcutanée est présent à raison de 15 % à 25 % du poids de la composition.

6. Composition pharmaceutique selon une des Revendications 1 à 5
**caractérisée en ce que** le solvant non aqueux physiologiquement acceptable est présent à raison de 44 % à 84,9 % du poids de la composition.

7. Composition pharmaceutique selon l'une quelconque des Revendications 1 à 6 **caractérisée en ce que** le polymère ou copolymère cellulosique est l'éthylcellulose, l'acétate butyrate de cellulose, l'acétate propionate de cellulose ou une hydroxypropylmethylcellulose greffée ou non.

8. Composition pharmaceutique selon l'une quelconque des Revendications 1 à 7 **caractérisée en ce que** le polymère ou copolymère cellulosique est l'éthylcellulose.

9. Composition pharmaceutique selon une des Revendications 1 à 8 **caractérisée en ce que** le principe actif est une substance soluble dans le solvant non aqueux physiologiquement acceptable choisie dans les groupes suivants :
un bronchodilatateur ; un diurétique ; un agent antibactérien, un agent antiacnéique ; un sédatif ou tranquilisant ; un psychostimulant ; un anxiolytique, une hormone ; un stéroïde androgénique ; un stéroïde oestrogénique ; un stéroïde progestatif ; une hormone thyroïdienne, un antipyrétique ; un analgésique narcotique ou un analgésique majeur ; un hypoglycémiant ; un antispasmodique ; un antitabagique ; un antimalarique ; un beta-bloquant ; un agent antiarthritique ; un agent anti-inflammatoire non stéroïdien, un agent antiostéoporotique ; un agent de blanchiment cutané ; un vasodilateur ; un antihypertenseur ; un antiparkinsonien ; un antimigraineux ; un agent contraceptif ; un antiulcéreux ; un anticancéreux ; un apport nutritionnel.

10. Composition pharmaceutique selon une des Revendications 1 à 9 **caractérisée en ce que** le principe actif est choisi parmi l'estradiol ; la sélégiline, l'ibuprofène, la clonidine, l'acétate de noréthindrone, la testostérone ou l'acide acétylsalicylique.

11. Composition pharmaceutique selon la Revendication 10, **caractérisée en ce que** l'estradiol est présent à raison de 0,5 % à 6 % du poids de la composition.

12. Composition pharmaceutique selon la Revendication 11, **caractérisée en ce que** l'estradiol est présent à raison de 1 % à 2 % du poids de la composition.

13. Composition pharmaceutique selon une des Revendications 1 à 9 **caractérisée en ce qu'**elle contient une association de principes actifs constituée d'un stéroïde progestatif et d'un stéroïde oestrogénique.

14. Composition pharmaceutique selon une des Revendications 1 à 13 **caractérisée en ce que** R₁ représente un groupement isopropyle, 2-éthyl-hexyle ou 1, 2-dihydroxy-éthyle.

15. Composition pharmaceutique selon la revendication 1 **caractérisée en ce que** le promoteur d'absorption transcutanée est choisi parmi les :
. 2-éthyl-hexanoate de 2-éthyl-hexyle
. myristate d'isopropyle
. myristate de l'éther monoéthylique du diéthylèneglycol
. palmitate d'isopropyle
. 2-octyldodécanol
. undécylénate de 2-éthyl-hexyle
. succinate de 2-éthyl-hexyle
. 12-hydroxy-stéarate de 2-éthyl-hexyle
. 12 acétoxy-stéarate de 2-éthyl-hexyle
. isostéarate de glycérol
. laurate d'hexyle.

16. Composition pharmaceutique selon la Revendication 1 ou 14 **caractérisée en ce que** le promoteur d'absorption transcutanée est le 2-éthyl-hexanoate de 2-éthyl-hexyle.

17. Composition pharmaceutique selon une des Revendications 1 à 16 **caractérisée en ce que** le solvant non aqueux physiologiquement acceptable est un composé de point d'ébullition inférieur à 100°C à pression atmosphérique.

18. Composition pharmaceutique selon la Revendication 17 **caractérisée en ce que** le composé de point d'ébullition inférieur à 100 °C est le dichlorométhane, l'éthanol, l'isopropanol ou l'acétate d'éthyle.

19. Composition pharmaceutique selon la Revendication 17 **caractérisée en ce que** le solvant non aqueux physiologiquement acceptable est l'éthanol.

20. Composition pharmaceutique selon la revendication 1 **caractérisée en ce que** :
. le polymère ou copolymère cellulosique est l'éthylcellulose
. le principe actif est l'estradiol
. le promoteur d'absorption transcutanée est le 2-éthyl-hexanoate de 2-éthyl-hexyle
. le solvant physiologiquement acceptable est l'éthanol.

21. Composition pharmaceutique selon la revendication 20 **caractérisée en ce qu'**elle consiste en :
. 3 % d'éthylcellulose ;
. 2 % d'estradiol ;
. 20 % de 2-éthyl-hexanoate de 2-éthyl-hexyle ; et
. 75 % d'éthanol.

22. Composition pharmaceutique selon la revendication 1 **caractérisée en ce qu'**elle consiste en :
. 5 % d'éthylcellulose ;
. 2 % d'estradiol ;
. 20 % de 2-éthyl-hexanoate de 2-éthyl-hexyle ; et
. 73 % d'éthanol.

23. Composition pharmaceutique selon la revendication 1 **caractérisée en ce qu'**elle consiste en :
. 5 % d'éthylcellulose ;
. 1 % d'estradiol ;
. 20 % de 2-éthyl-hexanoate de 2-éthyl-hexyle ; et
. 74 % d'éthanol.

24. Composition pharmaceutique selon la revendication 1 **caractérisée en ce qu'**elle consiste en :
. 2 % d'éthylcellulose ;
. 1 % d'estradiol ;
. 20 % de 2-éthyl-hexanoate de 2-éthyl-hexyle ; et
. 77 % d'éthanol.

25. Composition selon une des Revendications 1 à 24 **caractérisée en ce qu'**elle est destinée à être appliquée par pulvérisation directe sans l'aide de gaz propulseur comprimé ou liquéfié.

26. Composition selon une des Revendications 1 à 25 **caractérisée en ce qu'**elle est appliquée sur une zone cutanée de 10 à 40 cm².

27. Matrice pour composition pharmaceutique destinée à une administration transdermique **caractérisée en ce qu'**elle consiste en :
a) une matrice polymérique, pour le relargage d'un principe actif, capable de former un film souple après séchage, choisi parmi des polymères ou copolymères cellulosiques ;
b) 15 à 30 %, par rapport au poids de la composition, d'un promoteur d'absorption transcutanée du principe actif ;
c) un solvant non aqueux physiologiquement acceptable capable de dissoudre la matrice de relargage et le promoteur d'absorption transcutanée ainsi que de s'éliminer rapidement par évaporation au contact de la peau, le promoteur d'absorption transcutanée étant choisi parmi :
. un ester d'acide gras aliphatique soluble dans le solvant non aqueux physiologiquement acceptable et de formule générale : 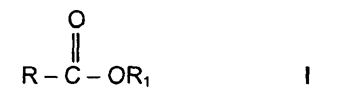 dans laquelle R représente un groupement alkyle ou alcényle, linéaire ou ramifié, en C₂-C₁₇ éventuellement substitué par un groupement hydroxyle, carboxylique ou acyloxy en C₁-C₄ et R₁ représente un groupement alkyle, linéaire ou ramifié, en C₃-C₈ éventuellement substitué par un ou deux groupements hydroxyles ou R₁ représente un groupement -CH₂-CH₂-O-(CH₂)₂-O-CH₂-CH₃, l'ester d'acide gras aliphatique comportant un minimum de 10 atomes de carbone et un maximum de 2 groupements hydroxyles
. un alcool gras aliphatique, soluble dans le solvant non aqueux physiologiquement acceptable de formule générale :
R₂-OH II
dans laquelle R₂ représente un groupement alkyle en C₁₀-C₂₀.

28. Matrice pour composition pharmaceutique selon la Revendication 27 **caractérisée en ce que** le polymère ou copolymère cellulosique est l'éthylcellulose, l'acétate butyrate de cellulose, l'acétate propionate de cellulose ou une hydroxypropylméthylcellulose greffée ou non.

29. Matrice pour composition pharmaceutique selon la Revendication 28 **caractérisée en ce que** le polymère ou copolymère cellulosique est l'éthylcellulose.

30. Matrice pour composition pharmaceutique selon l'une des Revendications 27 à 29 **caractérisée en ce que** le promoteur d"absorption transcutanée est choisi parmi les :
. 2-éthyl-hexanoate de 2-éthyl-hexyle
. myristate d'isopropyle
. myristate de l'éther monoéthylique du diéthylèneglycol
. palmitate d'isopropyle
. 2-octyldodécanol
. undécylénate de 2-éthyl-hexyle
. succinate de 2-éthyl-hexyle
. 12-hydroxy-stéarate de 2-éthyl-hexyle
. 12-acétoxy-stéarate de 2-éthyl-hexyle
. isostéarate de glycérol
. laurate d'hexyle.

31. Matrice pour composition pharmaceutique selon la Revendication 30 **caractérisée en ce que** le promoteur d'absorption transcutanée est le 2-éthyl-hexanoate de 2-éthyl-hexyle.

32. Matrice pour composition pharmaceutique selon une des Revendications 27 à 31 **caractérisée en ce que** le solvant non aqueux physiologiquement acceptable est un composé de point d'ébullition inférieur à 100°C à pression atmosphérique.

33. Matrice pour composition pharmaceutique selon la Revendication 32 **caractérisée en ce que** le composé de point d'ébullition inférieur à 100°C est le dichlorométhane, l'éthanol, l'isopropanol ou l'acétate d'éthyle.

34. Matrice pour composition transdermique selon la Revendication 32 **caractérisée en ce que** le composé de point d'ébullition inférieur à 100°C est l'éthanol.

35. Matrice pour composition pharmaceutique selon une des Revendications 27 à 34 **caractérisée en ce qu'**au sein de ladite composition pharmaceutique contenant le principe actif, la matrice de relargage représente au plus 6 %, le promoteur d'absorption transcutanée représente de 15 % à 30 % et le solvant non aqueux physiologiquement acceptable représente de 44 % à 84,9 %, ces pourcentages étant exprimés en poids de la composition pharmaceutique finale.

## Claims

1. Pharmaceutical composition for transdermal administration **characterised in that** it consists of:
a) a polymeric salting out matrix capable of forming a flexible film after drying, selected from among the cellulose polymers or copolymers;
b) an active principle;
c) 15 to 30%, based on the weight of the composition, of a promoter of the transcutaneous absorption of the active principle;
d) a non-aqueous physiologically acceptable solvent capable of dissolving the salting out matrix, the active principle and the transcutaneous absorption promoter and of being rapidly eliminated by evaporation on contact with the skin, the transcutaneous absorption promoter being selected from among:
• an aliphatic fatty acid ester soluble in the physiologically acceptable non-aqueous solvent and having the general formula: 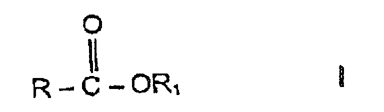 wherein R denotes a straight-chain or branched C₂-₁₇-alkyl or alkenyl group optionally substituted by a hydroxyl, carboxyl or C₁-₄-acyloxy group and R₁ denotes a straight-chain or branched C₃₋₈-alkyl group optionally substituted by one or two hydroxyl groups or R₁ denotes a group -CH₂-CH₂-O-(CH₂)₂-O-CH₂-CH₃, the aliphatic fatty acid ester comprising a minimum of 10 carbon atoms and a maximum of 2 hydroxyl groups
• an aliphatic fatty alcohol soluble in the physiologically acceptable non-aqueous solvent of the general formula:
R₂-OH II
wherein R₂ denotes a C₁₀₋₂₀-alkyl group.

2. Pharmaceutical composition according to claim 1, **characterised in that** the polymeric salting out matrix represents not more than 6% of the weight of the composition.

3. Pharmaceutical composition according to claim 2, **characterised in that** the polymeric salting out matrix is present in an amount of from 1% to 5% by weight of the composition.

4. Pharmaceutical composition according to one of claims 1 to 3, **characterised in that** the active principle is present in an amount of from 0.1% to 20% of the weight of the composition.

5. Pharmaceutical composition according to one of claims 1 to 4, **characterised in that** the transcutaneous absorption promoter is present in an amount of from 15% to 25% of the weight of the composition.

6. Pharmaceutical composition according to one of claims 1 to 5, **characterised in that** the physiologically acceptable non-aqueous solvent is present in an amount of from 44% to 84.9% by weight of the composition.

7. Pharmaceutical composition according to any one of claims 1 to 6, **characterised in that** the cellulose polymer or copolymer is ethyl cellulose, cellulose acetate butyrate, cellulose acetate propionate or a grafted or ungrafted hydroxypropylmethyl cellulose.

8. Pharmaceutical composition according to any one of claims 1 to 7, **characterised in that** the cellulose polymer or copolymer is ethyl cellulose.

9. Pharmaceutical composition according to one of claims 1 to 8, **characterised in that** the active principle is a substance which is soluble in the physiologically acceptable non-aqueous solvent selected from among the following groups:
a bronchodilator; a diuretic; an antibacterial agent, an anti-acne agent; a sedative or tranquilliser; a psychostimulant; an anxiolytic, a hormone, an androgenic steroid; an oestrogenic steroid; a progesterone steroid; a thyroid hormone, an antipyretic; a narcotic analgesic or a major analgesic; a hypoglycaemic agent; an antispasmodic agent; an anti-tobacco agent; an anti-malarial agent; a beta-blocker; an antiarthritic agent; a non-steroidal anti-inflammatory agent; an anti-osteoporotic agent; a skin whitening agent; a vasodilator; an antihypertensive; an anti-Parkinsonian; an antimigraine agent; a contraceptive agent; an antiulcer agent; an anti-cancer agent or a nutritional supplement.

10. Pharmaceutical composition according to one of claims 1 to 9, **characterised in that** the active principle is selected from among oestradiol; selegiline, ibuprofen, clonidine, norethindrone acetate, testosterone or acetylsalicylic acid.

11. Pharmaceutical composition according to claim 10, **characterised in that** the oestradiol is present in an amount of from 0.5% to 6% of the weight of the composition.

12. Pharmaceutical composition according to claim 11, **characterised in that** oestradiol is present in an amount of from 1 to 2% by weight of the composition.

13. Pharmaceutical composition according to one of claims 1 to 9, **characterised in that** it contains a combination of 10 active principles consisting of a progesterone steroid and an oestrogen steroid.

14. Pharmaceutical composition according to one of • claims 1 to 13, **characterised in that** R₁ denotes an isopropyl, 2-ethyl-hexyl or 1,2-dihydroxy-ethyl group.

15. Pharmaceutical composition according to claim 1, **characterised in that** the transcutaneous absorption promoter
is selected from among:
• 2-ethyl-hexyl-2-ethyl-hexanoate
• isopropyl myristate
• diethyleneglycol monoethylether myristate
• isopropyl palmitate
• 2-octyldodecanol
• 2-ethyl-hexyl undecylenate
• 2-ethyl-hexyl succinate
• 2-ethyl-hexyl-12-hydroxy-stearate
• 2-ethyl-hexyl-12-acetoxy-stearate
• glycerol isostearate
• hexyl laurate.

16. Pharmaceutical composition according to claim 1 or 14, **characterised in that** the transcutaneous absorption promoter is 2-ethyl-hexyl-2-ethylhexanoate.

17. Pharmaceutical composition according to one of claims 1 to 16, **characterised in that** the physiologically acceptable non-aqueous solvent is a compound with a boiling point below 100°C at atmospheric pressure.

18. Pharmaceutical composition according to claim 17, **characterised in that** the compound with a boiling point below 100°C is dichloromethane, ethanol, isopropanol or ethyl acetate.

19. Pharmaceutical composition according to claim 17, **characterised in that** the physiologically acceptable non-aqueous solvent is ethanol.

20. Pharmaceutical composition according to claim 1, **characterised in that**:
• the cellulose polymer or copolymer is ethyl cellulose
• the active principle is oestradiol
• the transcutaneous absorption promoter is 2-ethylhexyl-2-ethyl-hexanoate
• the physiologically acceptable solvent is ethanol.

21. Pharmaceutical composition according to claim 20, **characterised in that** it consists of:
• 3% ethyl cellulose;
• 2% oestradiol;
• 20% 2-ethyl-hexyl-2-ethyl-hexanoate; and
• 75% ethanol.

22. Pharmaceutical composition according to claim 1, **characterised in that** it consists of:
• 5% ethyl cellulose;
• 2% oestradiol;
• 20% 2-ethyl-hexyl-2-ethyl-hexanoate; and
• 73% ethanol.

23. Pharmaceutical composition according to claim 1, **characterised in that** it consists of:
• 5% ethyl cellulose;
• 1% oestradiol;
• 20% 2-ethyl-hexyl-2-ethyl-hexanoate; and
• 74% ethanol.

24. Pharmaceutical composition according to claim 1, **characterised in that** it consists of:
• 2% ethyl cellulose;
• 1% oestradiol;
• 20% 2-ethyl-hexyl-2-ethyl-hexanoate; and
• 77% ethanol.

25. Composition according to one of claims 1 to 24, **characterised in that** it is intended to be applied by direct spraying without the aid of a compressed or liquefied propellant gas.

26. Composition according to one of claims 1 to 25, **characterised in that** it is applied to an area of skin of from 10 to 40 cm².

27. Matrix for a pharmaceutical composition intended for transdermal administration, **characterised in that** it consists of:
a) a polymer matrix for salting out an active principle, capable of forming a flexible film after drying, selected from among the cellulose polymers or copolymers;
b) 15 to 30%, based on the weight of the composition, of a promoter of the transcutaneous absorption of the active principle;
c) a physiologically acceptable non-aqueous solvent capable of dissolving the salting out matrix and the transcutaneous absorption promoter and of being rapidly eliminated by evaporation in contact with the skin, the transcutaneous absorption promoter being selected from among:
• an aliphatic fatty acid ester soluble in the physiologically acceptable non-aqueous solvent and having the general formula: 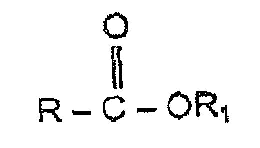 wherein R denotes a straight-chain or branched C₂₋₁₇-alkyl or alkenyl group optionally substituted by a hydroxyl, carboxyl or C₁₋₄-acyloxy group and R₁ denotes a straight-chain or branched C₃₋₈-alkyl group optionally substituted by one or two hydroxyl groups or R₁ denotes a group -CH₂-CH₂-O-(CH₂)₂-O-CH₂-CH₃, the aliphatic fatty acid ester comprising a minimum of 10 carbon atoms and a maximum of 2 hydroxyl groups
• an aliphatic fatty alcohol soluble in the physiologically acceptable non-aqueous solvent of the general formula:
R₂ - OH II
wherein R₂ denotes a C₁₀₋₂₀-alkyl group.

28. Matrix for a pharmaceutical composition according to claim 27, **characterised in that** the cellulose polymer or copolymer is ethyl cellulose, cellulose acetate butyrate, cellulose acetate propionate or a grafted or ungrafted hydroxypropylmethyl cellulose.

29. Matrix for a pharmaceutical composition according to claim 28, **characterised in that** the cellulose polymer or copolymer is ethyl cellulose.

30. Matrix for a pharmaceutical composition according to one of claims 27 to 29, **characterised in that** the transcutaneous absorption promoter is selected from among:
• 2-ethyl-hexyl-2-ethyl-hexanoate
• isopropyl myristate
• diethyleneglycol monoethylether myristate
• isopropyl palmitate 2-octyldodecanol
• 2-ethyl-hexyl undecylenate
• 2-ethyl-hexyl succinate
• 2-ethyl-hexyl-12-hydroxy-stearate
• 2-ethyl-hexyl-12-acetoxy-stearate
• glycerol isostearate
• hexyl laurate.

31. Matrix for a pharmaceutical composition according to claim 30, **characterised in that** the transcutaneous absorption promoter is 2-ethyl-hexyl-2-ethyl-hexanoate.

32. Matrix for a pharmaceutical composition according to one of claims 27 to 31, **characterised in that** the physiologically acceptable non-aqueous solvent is a compound with a boiling point below 100°C at atmospheric pressure.

33. Matrix for a pharmaceutical composition according to claim 32, **characterised in that** the compound with a boiling point below 100°C is dichloromethane, ethanol, isopropanol or ethyl acetate.

34. Matrix for a transdermal composition according to claim 32, **characterised in that** the compound with a boiling point below 100°C is ethanol.

35. Matrix for a pharmaceutical composition according to one of claims 27 to 34, **characterised in that**, within said pharmaceutical composition containing the active principle the salting out matrix constitutes at most 6%, the transcutaneous absorption promoter constitutes 15 to 30% and the physiologically acceptable non-aqueous solvent constitutes 44 to 84.9%, these percentages being expressed in terms of the weight of the final pharmaceutical composition.

## Patentansprüche

1. Pharmazeutische Zubereitung zur Verabreichung auf transdermalem Wege, **dadurch gekennzeichnet, daß** sie aus:
a) einer polymeren Freisetzungsmatrix, welche in der Lage ist, nach dem Trocknen einen weichen Film zu bilden, ausgewählt aus Cellulose-Polymeren oder -Copolymeren;
b) einem Wirkstoff;
c)15 bis 30 %, bezogen auf das Gewicht der Zubereitung, eines Promotors für die transkutane Absorption
des Wirkstoffs;
d) einem nichtwäßrigen, physiologisch verträglichen Lösungsmittel, welches in der Lage ist, die Freisetzungsmatrix, den Wirkstoff und den Promotor für die transkutane Absorption zu lösen und sich beim Kontakt mit der Haut schnell durch Verdampfen zu verflüchtigen,
besteht, wobei der Promotor für die transkutane Absorption ausgewählt ist aus:
• in dem nichtwäßrigen, physiologisch annehmbaren Lösungsmittel lösliche Ester von aliphatischen Fettsäuren der allgemeinen Formel: 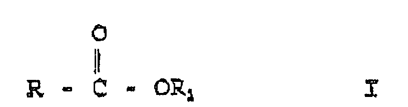 in der R eine geradkettige oder verzweigte C₂-C₁₇-Alkyl- oder C₂-C₁₇-Alkenylgruppe bedeutet, die gegebenenfalls durch eine Hydroxylgruppe, Carboxylgruppe oder C₁-C₄-Acyloxygruppe substituiert ist, und R₁ eine geradkettige oder verzweigte C₃-C₈-Alkylgruppe bedeutet, die gegebenenfalls durch eine oder zwei Hydroxylgruppen substituiert ist, oder R₁ eine Gruppe -CH₂-CH₂-O-(CH₂)₂-O-CH₂-CH₃ bedeutet, wobei der Ester der aliphatischen Fettsäure mindestens 10 Kohlenstoffatome und maximal 2 Hydroxylgruppen aufweist,
• in dem nichtwäßrigen und physiologisch verträglichen Lösungsmittel lösliche aliphatische Fettalkohole der allgemeinen Formel:
R₂ - OH II
in der R₂ eine C₁₀-C₂₀-Alkylgruppe bedeutet.

2. Pharmazeutische Zubereitung nach Anspruch 1, **dadurch gekennzeichnet, daß** die polymere Freisetzungsmatrix höchstens 6 % des Gewichts der Zubereitung ausmacht.

3. Pharmazeutische Zubereitung nach Anspruch 2, **dadurch gekennzeichnet, daß** die polymere Freisetzungsmatrix 1 % bis 5 % des Gewichts der Zubereitung ausmacht.

4. Pharmazeutische Zubereitung nach einem der Ansprüche 1 bis 3, **dadurch gekennzeichnet, daß** der Wirkstoff in einer Menge von 0,1 % bis 20 % des Gewichts der Zubereitung vorhanden ist.

5. Pharmazeutische Zubereitung nach einem der Ansprüche 1 bis 4, **dadurch gekennzeichnet, daß** der Promotor für die transkutane Absorption in einer Menge von 15 % bis 25 % des Gewichts der Zubereitung vorhanden ist.

6. Pharmazeutische Zubereitung nach einem der Ansprüche 1 bis 5, **dadurch gekennzeichnet, daß** das nichtwäßrige, physiologisch verträgliche Lösungsmittel in einer Menge von 44 % bis 84,9 % des Gewichts der Zubereitung vorhanden ist.

7. Pharmazeutische Zubereitung nach einem der Ansprüche 1 bis 6, **dadurch gekennzeichnet, daß** das Cellulose-Polymer oder -Copolymer Ethylcellulose, Celluloseacetat-butyrat, Celluloseacetat-propionat oder eine gegebenenfalls gepfropfte Hydroxypropylmethylcellulose ist.

8. Pharmazeutische Zubereitung nach einem der Ansprüche 1 bis 7, **dadurch gekennzeichnet, daß** das Cellulose-Polymer oder -Copolymer Ethylcellulose ist.

9. Pharmazeutische Zubereitung nach einem der Ansprüche 1 bis 8, **dadurch gekennzeichnet; daß** der Wirkstoff eine in dem nichtwäßrigen, physiologisch verträglichen Lösungsmittel lösliche Substanz ist ausgewählt aus den folgenden Gruppen:
Bronchodilatatoren, Diuretika, antibakterielle Mittel, Antiaknemittel, Sedativa oder Tranquilizer, psychostimulierende Mittel, anxiolytische Mittel, Hormone, androgene Steroide, östrogene Steroide, Progesteronsteroide, Schilddrüsenhormone, Antipyretika, narkotische Analgetika oder Allgemeinanalgetika; hypoglykämische Mittel, Antispasmodika, Mittel gegen Tabakmißbrauch, Antimalariamittel, Betablocker, Antiarthritismittel, nichtsteroidale an-tiinflammatorische Mittel, gegen Osteoporose wirkende Mittel, Hautbleichmittel, Vasodilatatoren, antihypertensive Mittel, Antiparkinsonmittel, Mittel gegen Migräne, Empfängsverhütungsmittel, Mittel gegen Geschwüre, Antikrebs-mittel und Nahrungsergänzungsmittel.

10. Pharmazeutische Zubereitung nach einem der Ansprüche 1 bis 9, **dadurch gekennzeichnet, daß** der Wirkstoff aus 55 Östradiol, Selegilin; Ibuprofen, Clonidin, Norethindrone-acetat, Testosteron und Acetylsalicylsäure ausgewählt ist.

11. Pharmazeutische Zubereitung nach Anspruch 10, **dadurch gekennzeichnet, daß** Östradiol in einer Menge von 0,5 % bis 6 % des Gewichts der Zubereitung vorhanden ist.

12. Pharmazeutische Zubereitung nach Anspruch 11, **dadurch gekennzeichnet, daß** Östradiol in einer Menge von 1 % bis 2 % des Gewichts der Zubereitung vorhanden ist.

13. Pharmazeutische Zubereitung nach einem der Ansprüche 1 bis 9, **dadurch gekennzeichnet, daß** sie eine Wirkstoffkombination enthält gebildet aus einem Progesteronsteroid und einem Östrogensteroid.

14. Pharmazeutische Zubereitung nach einem der Ansprüche 1 bis 13, **dadurch gekennzeichnet, daß** R₁ eine Isopropylgruppe, 2-Ethylhexylgruppe oder 1,2-Dihydroxy-ethylgruppe bedeutet.

15. Pharmazeutische Zubereitung nach Anspruch 1, **dadurch gekennzeichnet, daß** der Promotor für die transkutane Absorption ausgewählt ist aus:
• 2-Ethyl-hexyl-2-ethyl-hexanoat,
• Isopropylmyristat,
• Myristat des Monoethylethers von Diethylenglykol,
• Isopropylpalmitat,
• 2-Octyldodecanol,
• 2-Ethyl-hexyl-undecylenat,
• 2-Ethyl-hexyl-succinat,
• 2-Ethyl-hexyl-12-hydroxy-stearat,
• 2-Ethyl-hexyl-12-acetoxy-stearat,
• Glycerol-isostearat und
• Hexyllaurat.

16. Pharmazeutische Zubereitung nach Anspruch 1 oder 14, **dadurch gekennzeichnet, daß** der Promotor für die transkutane Absorption 2-Ethyl-hexyl-2-ethylhexanoat ist.

17. Pharmazeutische Zubereitung nach einem der Ansprüche 1 bis 16, **dadurch gekennzeichnet, daß** das nichtwäßrige, phyiologisch annehmbare Lösungsmittel eine Verbindung mit einem Siedepunkt von unterhalb 100°C bei Atmosphärendruck ist.

18. Pharmazeutische Zubereitung nach Anspruch 17, **dadurch gekennzeichnet, daß** die Verbindung mit einem Sie depunkt von unterhalb 100°C Dichlormethan, Ethanol, Isopropanol oder Ethylacetat ist.

19. Pharmazeutische Zubereitung nach Anspruch 17, **dadurch gekennzeichnet, daß** das nichtwäßrige, physiologisch annehmbare Lösungsmittel Ethanol ist.

20. Pharmazeutische Zubereitung nach Anspruch 1, **dadurch gekennzeichnet, daß**:
• das Cellulose-Polymer oder -Copolymer Ethylcellulose ist,
• der Wirkstoff Östradiol ist,
• der Promotor für die transkutane Absorption 2-Ethyl-hexyl-2-ethyl-hexanoat ist und
• das physiologisch annehmbare Lösungsmittel Ethanol ist.

21. Pharmazeutische Zubereitung nach Anspruch 20, **dadurch gekennzeichnet, daß** sie aus
• 3 % Ethylcellulose,
• 2 % Östradiol,
• 20 % 2-Ethyl-hexyl-2-ethyl-hexanoat und
• 75 % Ethanol besteht.

22. Pharmazeutische Zubereitung nach Anspruch 1, **dadurch gekennzeichnet, daß** sie aus
• 5 % Ethylcellulose,
• 2 % Östradiol,
• 20 % 2-Ethyl-hexyl-2-ethyl-hexanoat und
• 73 % Ethanol besteht.

23. Pharmazeutische Zubereitung nach Anspruch 1, **dadurch gekennzeichnet, daß** sie aus
• 5 % Ethylcellulose,
• 1 % Östradiol,
• 20 % 2-Ethyl-hexyl-2-ethyl-hexanoat und
• 74 % Ethanol besteht.

24. Pharmazeutische Zubereitung nach Anspruch 1, **dadurch gekennzeichnet, daß** sie aus
• 2 % Ethylcellulose,
• 1 % Östradiol,
• 20 % 2-Ethyl-hexyl-2-ethyl-hexanoat und
• 77 % Ethanol besteht.

25. Zubereitung nach einem der Ansprüche 1 bis 24, **dadurch gekennzeichnet, daß** sie durch direktes Aufsprühen ohne Hilfe eines verdichteten oder verflüssigten Treibgases verabreicht werden kann.

26. Zubereitung nach einem der Ansprüche 1 bis 25, **dadurch gekennzeichnet, daß** sie auf einen Hautbereich von 10 bis 40 cm² aufgebracht wird.

27. Matrix für eine pharmazeutische Zubereitung, die auf transdermalem Wege verabreicht werden soll, **dadurch gekennzeichnet, daß** sie aus:
a) einer polymeren Matrix für die Freisetzung eines Wirkstoffs, welche in der Lage ist, nach dem Trocknen einen weichen Film zu bilden, ausgewählt aus Cellulose-Polymeren oder -Copolymeren;
b) 15 bis 30 %, bezogen auf das Gewicht der Zubereitung, eines Promotors für die transkutane Absorption des Wirkstoffs;
c) einem nichtwäßrigen, physiologisch verträglichen Lösungsmittel, welches in der Lage ist, die Freisetzungsmatrix und den Promotor für die transkutane Absorption zu lösen und sich beim Kontakt mit der Haut schnell durch Verdampfen zu verflüchtigen, besteht, wobei der Promotor für die transkutane Absorption ausgewählt ist aus:
• in dem nichtwäßrigen, physiologisch annehmbaren Lösungsmittel lösliche Ester von aliphatischen Fettsäuren der allgemeinen Formel: 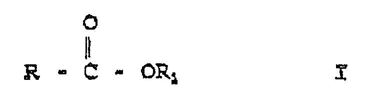 in der R eine geradkettige oder verzweigte C₂-C₁₇-Alkyl- oder C₂-C₁₇-Alkenylgruppe bedeutet, die gegebenenfalls durch eine Hydroxylgruppe, Carboxylgruppe oder C₁-C₄-Acyloxygruppe substituiert ist, und R₁ eine geradkettige oder verzweigte C₃-C₈-Alkylgruppe bedeutet, die gegebenenfalls durch eine oder zwei Hydroxylgruppen substituiert ist, oder R₁ eine Gruppe -CH₂-CH₂-O-(CH₂)₂-O-CH₂-CH₃ bedeutet,
wobei der Ester der aliphatischen Fettsäure mindestens 10 Kohlenstoffatome und maximal 2 Hydroxylgruppen aufweist,
• in dem nichtwäßrigen und physiologisch verträglichen Lösungsmittel lösliche aliphatische Fettalkohole der allgemeinen Formel:
R₂ - OH II
in der R₂ eine C₁₀-C₂₀-Alkylgruppe bedeutet.

28. Matrix für pharmazeutische Zubereitung nach Anspruch 27, **dadurch gekennzeichnet, daß** das Cellulose-Polymer oder -Copolymer Ethylcellulose, Cellulose-acetat-butyrat, Cellulose-acetatpropionat oder eine gegebenenfalls gepfropfte Hydroxypropylmethylcellulose ist.

29. Matrix für pharmazeutische Zubereitung nach Anspruch 28, **dadurch gekennzeichnet, daß** das Cellulose-Polymer oder -Copolymer Ethylcellulose ist.

30. Matrix für pharmazeutische Zubereitung nach einem der Ansprüche 27 bis 29, **dadurch gekennzeichnet, daß** der Promotor für die transkutane Absorption ausgewählt ist aus:
• 2-Ethyl-hexyl-2-ethyl-hexanoat,
• Isopropylmyristat,
• Myristat des Monoethylethers von Diethylenglykol,
• Isopropylpalmitat,
• 2-Octyldodecanol,
• 2-Ethyl-hexyl-undecylenat,
• 2-Ethyl-hexyl-succinat,
• 2-Ethyl-hexyl-12-hydroxy-stearat,
• 2-Ethyl-hexyl-12-acetoxy-stearat,
• Glycerol-isostearat und
• Hexyllaurat.

31. Matrix für pharmazeutische Zubereitung nach Anspruch 30, **dadurch gekennzeichnet, daß** der Promotor für die transkutane Absorption 2-Ethyl-hexyl-2-ethyl-hexanoat ist.

32. Matrix für pharmazeutische Zubereitung nach einem der Ansprüche 27 bis 31, **dadurch gekennzeichnet, daß** das nichtwäßrige, physiologisch annehmbare Lösungsmittel eine Verbindung mit einem Siedepunkt von unterhalb 100°C bei Atmosphärendruck ist.

33. Matrix für pharmazeutische Zubereitung nach Anspruch 32, **dadurch gekennzeichnet, daß** die Verbindung mit einem Siedepunkt von unterhalb 100°C Dichlormethan, Ethanol, Isopropanol oder Ethylacetat ist.

34. Matrix für transdermale Zubereitung nach Anspruch 32, **dadurch gekennzeichnet, daß** die Verbindung mit einem Siedepunkt von unterhalb 100°C Ethanol ist.

35. Matrix für pharmazeutische Zubereitung nach einem der Ansprüche 27 bis 34, **dadurch gekennzeichnet, daß** in der den Wirkstoff enthaltenden pharmazeutischen Zubereitung die Freisetzungsmatrix höchstens 6 %, der Promotor für die transkutane Absorption 15 % bis 30 % und das nichtwäßrige, physiologisch annehmbare Lösungsmittel 44 % bis 84,9 %, ausgedrückt als Prozentsatz des Gewichts der endgültigen pharmazeutischen Zubereitung, ausmachen.
